# EUROPEAN PATENT SPECIFICATION

(11) **EP 4 623 977 B1**
(45) Date of publication and mention of the grant of the patent: **21.10.2026**
(21) Application number: 25195472.3
(22) Date of filing: 03.07.2018
(51) Int. Cl.: A61M 11/00, A61M 15/08, A61M 16/14, A61M 16/16

(54) **GAS THERAPY SYSTEM FOR DELIVERY OF MEDICAMENT**
GASTHERAPIESYSTEM ZUR VERABREICHUNG EINES MEDIKAMENTS
SYSTÈME DE THÉRAPIE PAR GAZ POUR L'ADMINISTRATION D'UN MÉDICAMENT

(30) Priority: 04.07.2017 EP 17179658
(43) Date of publication of application: 01.10.2025
(62) Document number(s) of the earlier application(s) in accordance with Art. 76 EPC: 23213965.9 / 4 309 718; 23162159.0 / 4 215 233; 18734571.5 / 3 648 817
(73) Proprietor: Stamford Devices Limited, Dangan, Galway (IE)
(72) Inventor: MACLOUGHLIN, Ronan, Galway (IE); PORTER, Shaun, Galway (IE); DUFFY, Conor, Galway (IE); POWER, John, Galway (IE)
(74) Representative: Weldon O'Brien Ltd.

(56) References cited:
- WO-A1-2013/163527
- WO-A1-2015/155342
- WO-A2-2007/008825

## Description

### INTRODUCTION

### Field of the Invention

The invention relates to a gas therapy system for use where patients require respiratory support and/or supplemental oxygen. An example of such is a high flow nasal therapy (HFNT) system, in which the flow is directed through a nasal cannula.

Gas therapy such as high flow gas therapy is a growing therapy providing respiratory support to patients requiring breathing support and/or supplemental oxygen, in which delivered flow rates may be high, up to 60L/min in many cases. The suggested mechanisms by which such systems provide respiratory support include:
- reduction of dead space, and
- positive airway pressure promoting more controlled breathing

However the use of these relatively high flows does not facilitate optimal aerosol delivery and, low single figure deposition percentage efficiencies have been reported. In the past, the approach has often been complete removal of the patient from respiratory support in order to administer aerosol.

WO2015/155342 (Stamford Devices Ltd) describes a HFNT system in which there is increased aerosol delivery during patient inhalation, and reduction of gas flow during aerosol therapy.

The invention is directed towards achieving improved delivery of aerosol during high flow therapy.

### SUMMARY OF THE INVENTION

The invention provides a gas therapy device as set out in claim1, and optional features in claims 2 to 14.

### DETAILED DESCRIPTION OF THE technology

### Brief Description of the Drawings

The technology will be more clearly understood from the following description of some embodiments thereof, given by way of example only with reference to the accompanying drawings in which:-
Fig. 1(a) is a diagram showing a HFNT system of the technology connected to a patient;
and Fig. 1(b) is a view showing the physical arrangement and an alternative arrangement;
Figs. 2 to 4 are plots showing gas flow rate versus time for examples of use of the system;
Fig. 5 is a flow diagram for operation of the system in one example; and Fig. 6 is a series of plots illustrating relationships between patient breathing, gas flow, and aerosolization;
Fig. 7 is a plot of tracheal dose vs aerosol interval duration for systems where the nebulizer is next to the humidifier and next to the cannula as shown in Fig. 1(b); and
Fig. 8 is a pair of plots illustrating tracheal dose vs. aerosol interval duration with and without phasic delivery according to patient breath monitoring.

### Description of the Embodiments

The technology is described below for a high flow nasal therapy ("HFNT") system, but it applies equally to other gas therapy systems especially including high flow systems such as CPAP, BiPAP, and resuscitation. The system performs integrated cycling in which a nebulizer and a gas flow are cycled in an inverse manner. For example, at higher flows of over 20LPM (litres per minute) no aerosol will be generated, however, when the nebulizer is in operation the system will switch to a low flow of for example 10LPM to facilitate greater aerosol deposition, while the nebulizer will be operating at maximum output. The gas flow will preferably not decrease to zero so as to reduce but not eliminate the respiratory support.

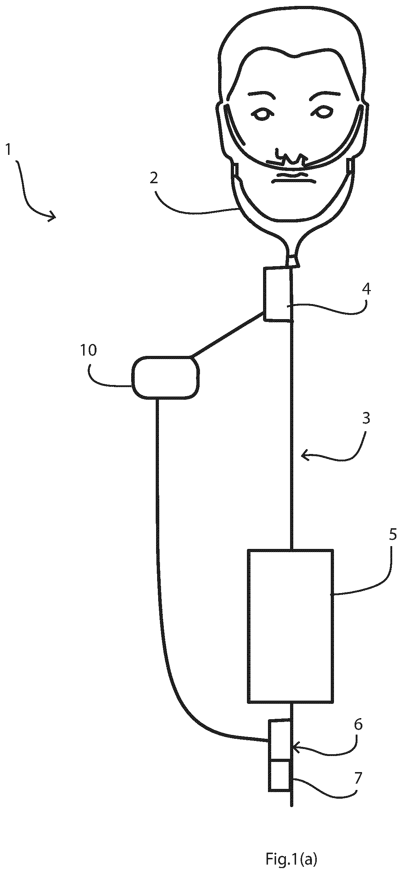

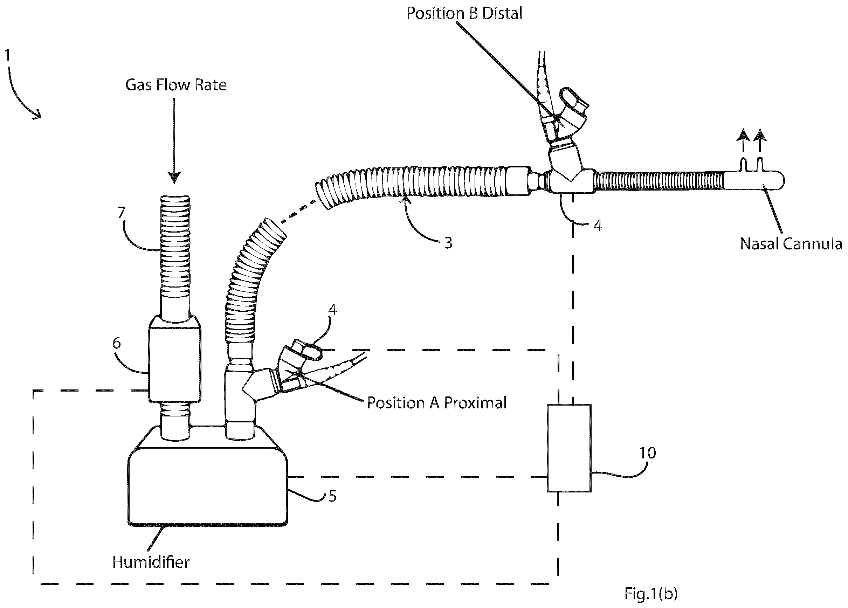

Referring to Figs. 1(a) and 1(b) a HFNT system 1 comprises a nasal cannula 2 supplied by a flow line 3 including a nebulizer 4 and a heated humidifier 5. The line 3 has an air inlet 7 alongside which there is a flow meter/regulator 6. The gas source could be supplied from a gas blender, directly connected to medical gas supply or from a flow blower or turbine. Fig. 1(a) shows in diagrammatic terms the arrangement where the nebulizer is near a cannula. Fig. 1(b) shows the physical arrangement and also shows an alternative use in which the nebulizer 4 is close to the humidifier 5 (Position A, Proximal). This shows versatility of the system in use, and the description below describes the aspects which arise from these different nebulizer positions.

A programmed controller 10 is linked with the nebulizer 4, the air inlet 7, and the flow meter/regulator 6 to perform dynamic real time control, as described below. A user interface may be incorporated as part of the controller 10, or be linked by cable or wireless such as Bluetooth. Unidirectional flow is from the air inlet 7 towards the nasal interface 2 via a single main limb 3 that may split into two sides at the nasal interface 2.

The system control unit 10 houses a digital processor programmed to control a flow blower or turbine linked with the inlet 7, to, control the gas flow from the gas inlet 7 and into the humidifier. Hence, the controller 10 can control the rate of gas flow, and this is achieved with short ramp times to reach the target rates, typically in the range of 50ms to 100ms. The majority of treatment time is with gas flow at the higher rate (e.g. 60 LPM), and this flow is interrupted at intervals with lower flow rates (e.g. 10 LPM, 30LPM). Where patients are too ill to be able to cope with a reduction to the order of 10LPM, the lower level may for example be higher such as a value in the range of 20LPM to 40LPM.

There is aerosol delivery during these intervals. The intervals are preferably triggered at target times according to the extent of required aerosol therapy. Preferably, the intervals are automatically chosen to coincide with patient inhalation.

It is envisaged that in some cases the cycle will be 60 seconds, with one interval in each such cycle for a minority of the 60 seconds.

Flow control may alternatively be achieved by restriction of a flow orifice by electronic control such as a solenoid valve. Such electronic control is also capable of response times in the region of 50ms to 100ms.

The system may include a flow sensor linked with the controller and being at any location between the gas source and the cannula, for detection of the gas flow from the gas source. This may be used by the controller in its operations to automatically trigger aerosol delivery in addition to or instead of using the valve control signals.

The controller 10 is also connected to the aerosol generator 4 for on/off actuation.

The aerosol delivery is controlled to turn on or turn off in a duration under 25ms, preferably under 10ms. The gas flow is controlled with a ramp up or a ramp down duration of under 100ms, preferably under 50ms.

During high flow therapy at higher gas flow rates (range 10 to 60 LPM for example), the aerosol generator 4 operation is controlled to stop or reduce rate of aerosol generation.

During high flow therapy at lower gas flow rates (range 1 to 10 LPM for example), the aerosol generator operation is controlled to maximise aerosol output.

If the user selects a suboptimal gas flow rate (with regards to aerosol delivery) during periods in which the aerosol generator 4 is in operation, the controller 10 acts to control the gas flow rate.

The system maintains respiratory support with the prescribed upper gas flow rate for the majority of time, but with intermittent short periods of respiratory support with lower gas flow rates. It is during these intermittent, lower gas flow rate intervals only that aerosol is generated.

In the past, the approach has often been complete removal of the patient from high flow respiratory support to administer aerosol using a handheld, or other aerosol delivery system. The invention is therefore very advantageous by allowing for the maintenance of respiratory support during aerosol delivery and mitigating the risk of potential de-recruitment of the lung during periods of low or no gas flow during aerosol therapy.

In other embodiments the system also includes sensors such as sensors to detect patient breathing cycles, as described in more detail below. There may be sensors to detect patient physiological parameters which indicate extent of real time need for gas therapy and/or aerosol therapy. Delivery of aerosol on a breath-activated basis is referred to as "phasic delivery". The vast majority of aerosol being delivery during inhalation, for optimum tracheal dose efficiency.

In overview, the controller switches between upper and lower levels of gas flow rates, and activates aerosol delivery during some or all of the time that the gas flow is at the lower rate. This is in some embodiments based on time cycles, such as a cycle of 45 seconds, 30 seconds being upper rate and 15 seconds being lower rate. There may be dynamic changes to this depending on user inputs at the UI and/or sensed conditions such as a pressure or humidity sensor detecting patient inhalation and/or sensors to detect a patient condition indicating when gas flow needs to switch to the upper rate. The controller may apply a completely manual override in certain situations.

**Table 1: Control algorithm calculations.**

| | **Fluidic volume (mL)** | | | | | |
|---|---|---|---|---|---|---|
| **Example Location of aerosol generator** | **Humidifier** | **Tubing** | **Patient interface** | **Total Volume (Litres)** | **Gas Flow Rate (LPM)** | **Time for aerosol to travel from aerosol generator to patient (secs)** |
| Dry side of humidifier | 150 | 250 | 5 | 0.405 | 60 | 0.41 |
| | 150 | 250 | 5 | 0.405 | 10 | 2.43 |
| At patient interface | n/a | n/a | 5 | 0.005 | 60 | 0.01 |
| | n/a | n/a | 5 | 0.005 | 10 | 0.03 |

Examples of calculations used in the control of the gas flow and aerosol generation are presented in Table 1. These worked examples illustrate the differences in transit time through the system relative to the position of the aerosol generator 4 and gas flow rate.

The intervals for aerosol delivery (with lower gas flow) may be as short as only 2 minutes per hour or less, depending on the clinical situation. The nebuliser duty cycle may be for example 5%, 10%, or 20%, and in general it is preferred if it is in the range of about 2% to 50 % for optimum dose efficiency.

Further, the aerosol generator shall not affect the flows or pressures being applied to the patient. For example, preferred aerosol generators include, but do are not limited to, static or actively vibrating mesh nebulisers, surface acoustic wave aerosol generators, Fourier horn-type aerosol generators. An example of a non-preferred aerosol generator is the jet-type nebulisers that require a driving gas for operation.

The following describes the invention in more detail.

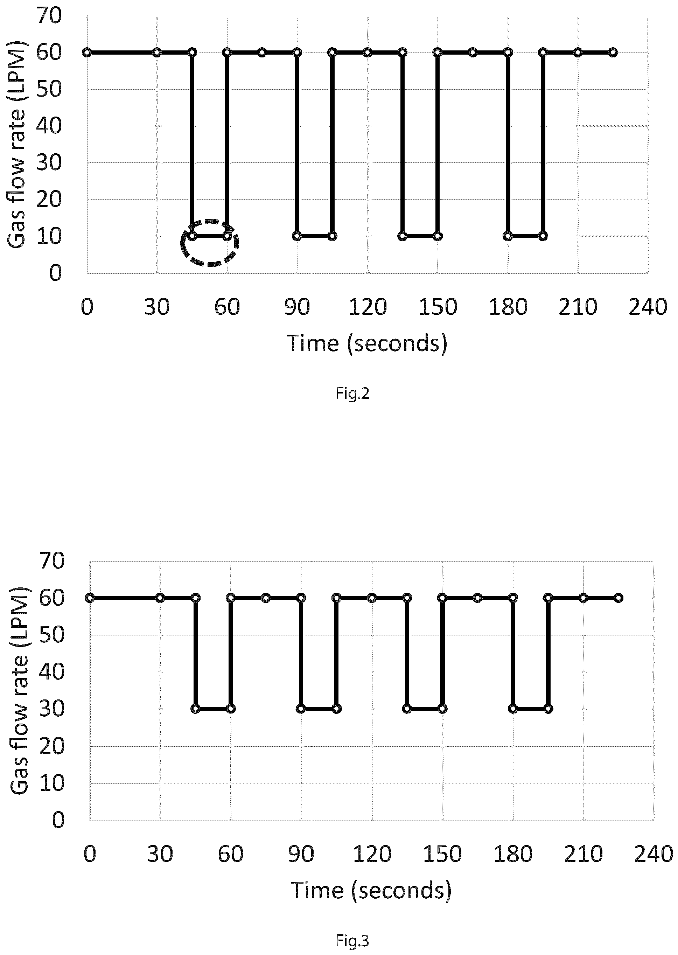

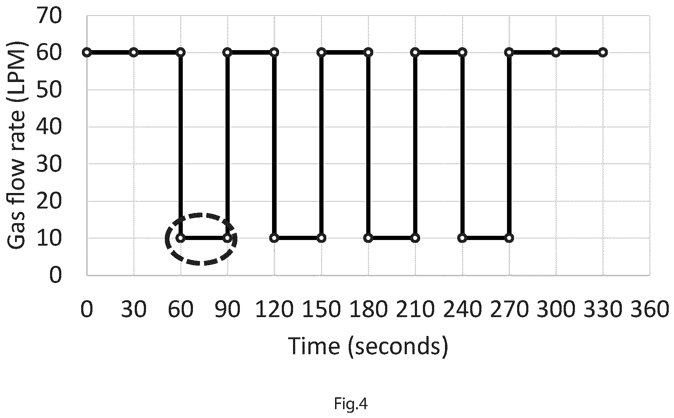

Fig. 2 is an illustration of gas flow rate delivered to the patient at 60 LPM and 10 LPM, with a 60LPM period of 30 s (seconds) and a 10 LPM interval of 15 s making up each cycle. Fig. 3 shows a control scheme in which the switching is for the same time periods, but the lower rate is 30LPM. Fig. 4 shows a scheme in which the periods are equal, 30 s, and the rates are 60LPM and 10LPM.

The optimal duration of aerosol generation and the associated lower gas flow rate (in minutes or seconds), is in one example depending on, among other parameters, output of a calculation of internal circuit volume between the nebuliser 4 and the end of the nasal interface 2 divided by the gas flow rate. Sufficient time is required for aerosol generation, transit through the internal tubes and delivery beyond the nasal interface. The relative periods of optimal and suboptimal gas flow rates may change, and the examples provided herein are for illustration purposes only.

As shown in Fig. 1(b) the aerosol generator may be placed: (a) at the nasal interface, (b) between the nasal interface and the circuit tubing, or (c) attached to the humidifier (which may or may not be heated) and/or the gas flow generator/source.

The tracheal dose, i.e. amount of aerosol inhaled by a simulated patient and captured at the level of the trachea, characterised across low and high gas flow rate settings commonly used in Adults, and the improved method are presented in Table 2 below.

### TRACHEAL DOSE

**Table 2: Tracheal dose.**

| | | **Tracheal Dose (%)** | |
|---|---|---|---|
| | **Gas Flow (LPM)** | **3.3µm Droplet Size** | **4.5µm Droplet Size** |
| | 60 | 3.45 ± 0.47 | 2.15 ± 0.13 |
| Dosing examples | 30 seconds of 60LPM + 15 seconds of 30LPM | 10.43 ± 0.88 | 7.98 ± 0.69 |
| | 30 seconds of 60LPM + 15 seconds of 10LPM | 31.74 ± 1.15 | 25.15 ± 0.52 |
| | 30 seconds of 30LPM + 15 seconds of 10LPM | 25.86 ± 2.05 | 19.44 ± 0.93 |

The tracheal dose is the drug mass delivered to the level of the trachea in a bench model of a breathing adult patient (15BPM (breaths per minute), Inspiratory : Expiratory (I:E) ratio of 1:1, and a tidal volume (Vt) of 500mL), expressed as a percentage of the nominal dose placed in the aerosol generator medication cup.

In the examples above, the higher gas flow was run for 30 seconds and the lower gas flow was run for 15 seconds. These durations are to be considered an example of the available options. As described in more detail below, the control scheme may be based on different time periods or may be based on active sensing of a patient, such as according to breaths.

In Table 2 it is noted that decreasing gas flow rate facilitates an increase in tracheal dose. This is however at the considerable expense of much reduced gas flow therapy, which in many clinical situations is not possible without risk to the patient. It is also noted that a 3.3µm (Dv50) droplet size (emitted from the aerosol generator) facilitates an increase in tracheal dose over a 4.5µm (Dv50) droplet size.

Switching of gas flow between upper and lower rate levels and generation of aerosol only during those lower gas flows, facilitates a higher tracheal dose than that recorded for the high gas flow rate. Moreover it also, surprisingly, achieves a higher tracheal dose than would be the case for a continuous lower gas flow rate level. The dose proportion delivered is about 26% when the gas flow rate is continuously 10LPM, whereas when it is switched between 60 LPM and 10 LPM in cycles with periods of 30 s and 15 s respectively the proportion of dose delivered is about 32%. Again, lower droplet size facilitates a greater tracheal dose.

Whilst the differential between reducing the flow from 60 to 30 LPM is not as great as reducing flow from 60LPM to 10LPM, this still demonstrates that there are benefits even while reducing the flow from the high value of 60LPM to a lower flow.

Also, it is envisaged that with lower sized droplets, e.g. in the range of 0.5 µm to 4.0 µm (e.g. 3.3 µm) as compared to about 4.5 µm there may be significant benefits with respect to delivered dose.

In another embodiment, the system performs breath control of aerosol delivery, in which the aerosol generator is only active in periods of inhalation during which concurrent reduction in gas flow is imposed. Therefore, aerosolising during the exhalation phase is avoided, reducing possible drug wastage. Another aspect is that higher gas flow rates tend to reduce the number of breaths per minute and so this may be a significant source of delivery variation addressed with breath control. In one example control scheme, the controller activates the nebulizer only when both the assigned time period (such as 15 s in a 45 s cycle) is reached and there is inhalation. In another example, the controller activates the nebulizer and reduces gas flow only during inhalation in a sub-set of the total inhalations, such as every second inhalation or every third inhalation. Inhalation is preferably sensed in real time by pressure sensors in the cannula or the limb leading to the cannula. The sensor may detect thoracic or abdominal movement resultant from respiratory movements, such sensors include but are not limited to: Graseby respiration sensor, ultra-wideband, impedance plethysmography, respiratory inductance plethysmography or elastomeric plethysmography device.

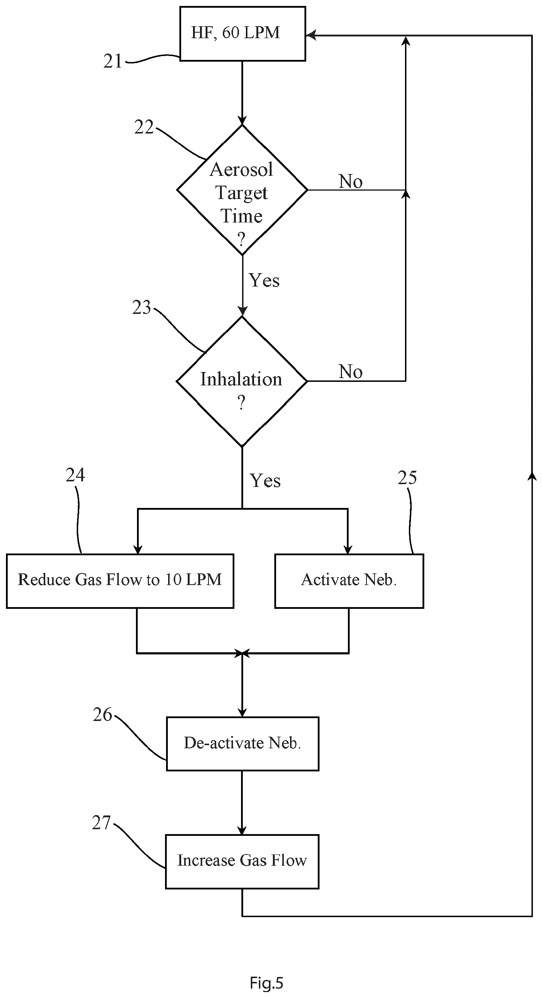

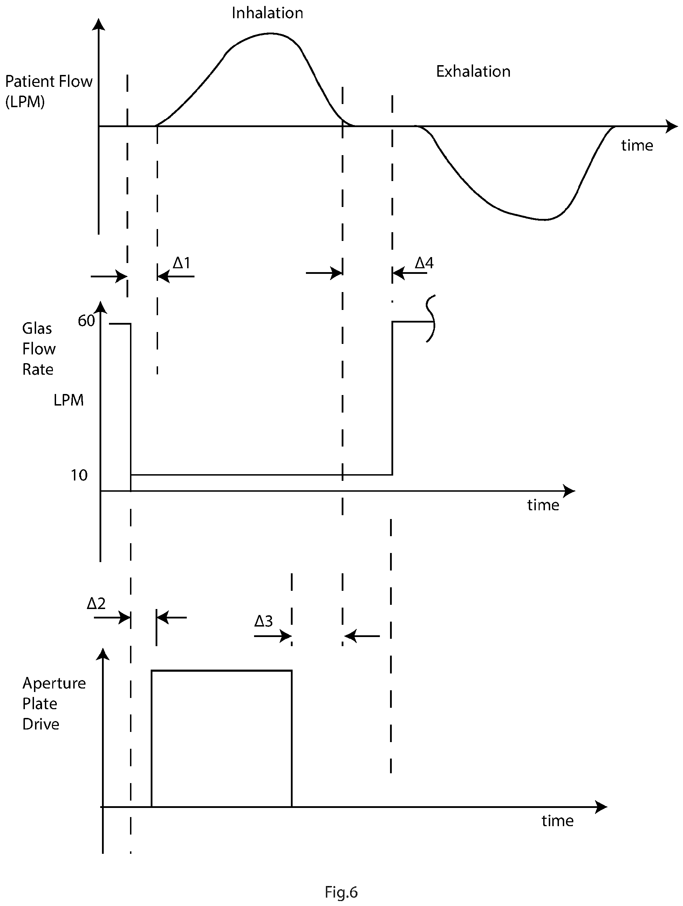

Referring to Figs. 5 and 6 in a method 20 performed by the digital processor of the controller 10 there is aerosol generation only when a target aerosolization time has arrived and there is patient inhalation. Step 21 represents the normal high gas flow rate level of 60LPM for patient treatment to ensure that there is sufficient delivery of oxygen to the lungs, clearance of the4 dead space of CO2 and maintenance of airway pressure.. Step 22 indicates the monitoring of time since start of treatment when it is preferred that aerosol be delivered, according to clinical requirements. If so, then the controller determines in step 23 according to the breathing sensors if there is inhalation. If so, the controller both reduces gas flows to only 10LPM in step 24 and activates the nebulizer in step 25. In this example there is phasic delivery with aerosolization only when there is patient inhalation. However, in other examples this is not essential.

These aspects are illustrated in Fig. 6. The top plot shows detected inhalation, represented as positive flow and exhalation as negative flow (could be opposite). Below that, the plot illustrates gas flow dropping from 60LPM to 10LPM as inhalation begins.

There is a small extent of advance dropping of the gas flow (Δ1) due to inhalation being predicted. The prediction may be based on the detected breathing pattern, with a predicted "breaths per minute" (BPM) rate. For example, if there are 10 breaths per minute the cycle time is 6 seconds, indicating an inspiration time of 3 seconds and a dwell time before exhalation.

Then, as shown in the bottom plot aerosol delivery is activated after a short delay of Δ2 from gas flow reduction. Aerosol delivery remains for a time direction well within the inhalation duration, ending Δ3 before end of inhalation. Then, after a delay of Δ4 from end of inhalation the gas flow returns to 60LPM, thus ending the interval.

Δ1 is not essential, but is preferred in order to ensure that the conditions are correct for time duration of 0.01 s to 3.0 s for aerosolization.

Also, Δ2 is not essential, but is preferred to ensure that optimum conditions exist for aerosolization. It is preferably short, in a range of about 0.01 s to 3.0 s. The value of Δ2 can be set according to desired penetration of the medicament. For example it can be very short or non-existent for particular targeting of the peripheral airways. It can be longer where the target is the conducting airway region, and it can be long if the target is the upper airway region.

Δ3 is a delay between end of aerosolization and end of inhalation. It may have a value in the range of 0.01 s to 3.0 s for the reasons given above for Δ2.

Δ4 depends on the length of dwell between inhalation and exhalation. It is preferred to have this delay short enough in order to avoid low flow during exhalation.

In other embodiments, the sensor includes a gas or volatile sensing device. Gases being detected may include, but are not limited to; carbon dioxide, a tracer gas/volatile delivered in tandem with the high flow, and nitric oxide.

The upper level gas flow duration may be from about 70% to about 99% of each cycle. For example, for a 5 sec cycle at 80% this equates to 4 sec period with upper level gas flow and no aerosol, and 1 second with lower gas flow rate and with aerosol delivery. It is preferred that the duty cycle for upper rate flow to lower level flow is 65% to 35%.

In a further case the system performs modulation of oxygen supplied during the lower gas flow rates, i.e. if the patient requires X % oxygen and that is delivered at 60LPM, then 10LPM gas flow will deliver less oxygen. Therefore increasing oxygen concentration in the gas flow during the lower gas flow rate interval may act to maintain the consistency of blood oxygenation. To achieve this, the system may include a real time pulse oximeter, an oxygen regulator, and/or a blender. In many cases the oxygen concentration is as high as 95%, and so there is little scope to increase it in the intervals. However, the benefit of increasing the oxygen concentration of the gas mix supplied during the intervals (air and oxygen, or Helium and oxygen) is more pronounced where the concentration at the higher flow rate level is less, say in the range of 50% to 80%. In this aspect the oxygen concentration control may be at least partly in response to detected SpO₂ levels.

Preferably, the controller executes an algorithm to calculate a value for time aerosol takes to move from the aerosol generator to the end of cannula, and to add sufficient time for a breath. This will help determine the length of time for each period of the lower gas flow rate. Table 1 above gives exemplary data on which this can be based.

In many cases the intervals of aerosol delivery are not every breath. For example, the system may be set to deliver aerosol once every X number of minutes. X is preferably determined according to the risk of lung de-recruitment. Preferably, the controller includes stored patient-specific settings which it uses to automatically determine frequency of aerosol delivery.

The system may in some examples generate a bolus of aerosol within the flow line for optimal aerosol delivery during the lower gas flow rates. This may be achieved by the gas flow line being divided into two limbs, one for aerosol delivery and the other for only gas flow. The bolus of aerosol is built up by operation of the nebulizer while the aerosol limb is closed, and this limb is opened during lower level gas flow.

In other examples, the controller is configured to manage flows according to inertial potential of the aerosol.

The volume of aerosol losses within the circuit and the consequent emitted dose, available for inhalation by the patient, is controlled by the aerosol droplet or particle size produced by the aerosol generator and the relative density of aerosol droplets or particles in the gas flow. Specifically, droplets or particles are smaller than the cut-off size imposed by the patient interface, e.g. nasal cannula size combined with the chosen gas flow or range of gas flow rates and the output from the aerosol generator is controlled such that the risk of inertial impaction within the circuit tubing is reduced.

The advantage of both droplet size and nebulizer duty cycle is reduced at higher gas flow rates. Therefore the combination of lower gas flow rate with lower droplet or particle size and lower nebulizer duty cycle provides major benefits. For example, it is preferred that the droplet size be in the range of 1.0 µm to 4.0 µm and that the nebulizer duty cycle be in the range of 2% to 20%.

For example 5% duty cycle may be 5 microseconds ON, 95 microseconds OFF; 10% duty cycle may be 10 microseconds ON, 90 microseconds OFF; and 20% duty cycle may be 20 microseconds ON, 80 microseconds OFF.

In other examples, assuming a range of 5 to 50 breaths per minute, this means that at 5BPM, each breath cycle will be 12 seconds in duration. ~ 50BPM, each breath will be 1.2 seconds long. If the system does not utilise breath controlled aerosol delivery, aerosol ON for 15 seconds will align with approximately 1.25 breaths for 5 BPM and 12.5 breaths for 50BPM. If the system does utilise breath controlled aerosol delivery the I:E ratio may be as much as 1:5:
@5BPM = 12 seconds => 2 seconds inhale
@50BPM = 1.2 seconds => 0.20 seconds inhale

With phasic (breath-actuated) delivery the duty cycle is variable, being responsive to current patient breathing pattern.

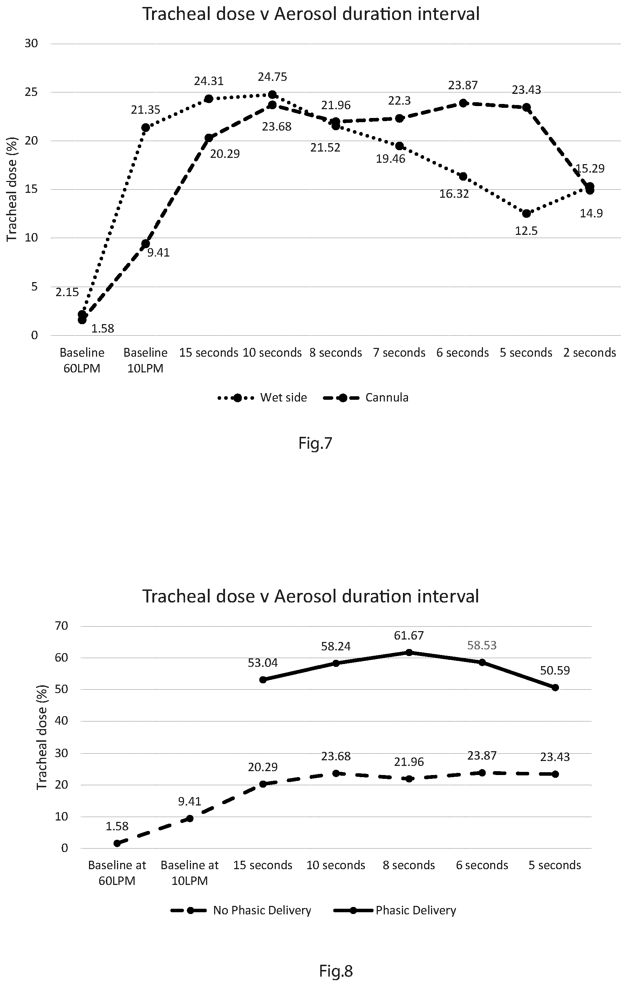

Fig. 7 shows test results for tracheal dose for the two possible set-ups shown in Fig. 1(b) - "Wet-side" just downstream and next to the humidifier, (Position A, Proximal), and "Cannula" (Position B, Distal). There is no breath detection control in these tests.

### Wet Side

The "baseline" tracheal dose (when the gas flow and aerosol generation remains constant) is 21% for 10LPM and 2% for 60LPM. Unsurprisingly the efficiency is very low for continuous 60LMM gas flow, which is part of the problem addressed by the invention. The tracheal dose improves considerably when the flow rate is down to 10LPM, however this is not always practical because the higher rates are needed for patent respiratory support.

With intervals as described for operation of the controller the dosage results remain good and indeed improve for intervals of between 15 secs and about 7 secs. However, for shorter intervals the dosage results fall off.

### Cannula Side

Again, dosage is very poor for the baselines, in fact worse here for the 10LPM continuous delivery. An important aspect is that the delivered dose does not drop off as the intervals decrease - still about 23% for intervals of 5 secs. It only drops significantly towards shorter intervals of about 2 seconds.

The data plotted in Fig. 7 is given below in Tables 3 and 4, in which "P-Value" is a unit-less representation of statistical significance of the difference between the columns, in which the lower the P-value the more significant it is, and a value of less than 0.5 is considered significant.

**Table 3**

| | **Tracheal dose (%) nebuliser at wet side** | **Tracheal dose (%) nebuliser at nasal cannula** |
|---|---|---|
| **Baseline at 10LPM** | 21.35 ± 0.23 | 9.41 ± 0.49 |
| **Baseline at 60LPM** | 2.15 ± 0.13 | 1.58 ± 0.12 |

**Table 4**

| **Aerosol duration at 10LPM** | **Tracheal dose (%) nebuliser at wet side** | **Tracheal dose (%) nebuliser at nasal cannula** | **P-value** |
|---|---|---|---|
| **15 seconds** | 24.31 ± 0.69 | 20.29 ± 0.70 | 0.01 |
| **10 seconds** | 24.76 ± 2.52 | 23.68 ± 1.27 | 0.72 |
| **8 seconds** | 21.52 ± 0.42 | 21.96 ± 1.06 | 0.71 |
| **7 seconds** | 19.46 ± 0.66 | 22.30 ± 2.16 | 0.26 |
| **6 seconds** | 16.32 ± 0.78 | 23.87 ± 2.14 | 0.03 |
| **5 seconds** | 12.50 ± 1.17 | 23.43 ± 0.47 | 0.00 |
| **2 seconds** | 15.30 ± 0.92 | 14.90 ± 0.93 | 0.78 |

If the controller processor is programmed as shown in Fig. 5 to provide phasic delivery (upon detected inhalation) the tracheal dose results improve significantly, as shown in Fig. 8 and in Table 5 below. For all measurements the nebulizer was at the cannula (Position B, Distal in Fig. 1(b)).

**Table 5**

| **Aerosol duration interval at 10LPM** | No **Phasic Delivery** | **Phasic Delivery** | **P-value** |
|---|---|---|---|
| **15 seconds** | 20.29 ± 0.70 | 53.04 ± 11.38 | 0.01 |
| **10 seconds** | 23.68 ± 1.27 | 58.24 ± 6.78 | 0.00 |
| **8 seconds** | 21.96 ± 1.06 | 61.67 ± 5.15 | 0.00 |
| **6 seconds** | 23.87 ± 2.14 | 48.82 ± 8.51 | 0.01 |
| **5 seconds** | 23.43 ± 0.47 | 50.59 ± 6.73 | 0.00 |

In this case the dosage is in the range of about 50% to 60% for intervals of 15 s to 5 s, with high flow at 60LPM for 30 s between each dosage interval.

### Effect of nebuliser position on aerosol efficiency during phasic delivery

Efficient and less efficient aerosol durations were selected; 8 seconds and 5 seconds respectively. In order to compare the effect of nebuliser position during phasic delivery, the nebuliser was positioned at the wet side of the humidifier for 8 second and 5 second aerosol durations. Results are outlined in Table 6.

**Table 6**

| **Aerosol duration at 10LPM** | **Nebuliser at cannula** | **Nebuliser at wet side** | **P-value** |
|---|---|---|---|
| **8 seconds** | 61.67 ± 5.15 | 8.33 ± 2.00 | 0.003 |
| **5 seconds** | 50.59 ± 6.73 | 6.96 ± 2.00 | 0.004 |

Table 6 shows tracheal dose (%) at the 8-second and 5-second aerosol duration settings when the nebuliser was placed at the wet side of the humidifier and at the nasal cannula. P-values are included to indicate significance. P-values of < 0.05 are considered statistically significant.

### Effect of droplet size on aerosol efficiency during phasic delivery

The preferred range for droplet size is approximately 1 µm to 10 µm.

Efficient and less efficient aerosol durations were selected; 8 seconds and 5 seconds respectively. In order to compare the effect of droplet size during phasic delivery, a nebuliser of low VMD was tested for the 8 second and 5 second aerosol durations. Results are outlined in Table 7 below.

**Table 7**

| **Aerosol duration at 10LPM** | **Standard VMD** | **Low VMD** | **P-value** |
|---|---|---|---|
| **8 seconds** | 61.67 ± 5.15 | 61.96 ± 4.20 | 0.94 |
| **5 seconds** | 50.59 ± 6.73 | 54.90 ± 9.56 | 0.56 |

Table 7 shows tracheal dose (%) at the 8 second and 5 second aerosol duration settings. P-values are included to indicate significance. P-values of < 0.05 are considered statistically significant.

In summary, tracheal dose was assessed during HFNT at the following gas flow rates: 60LPM for 30 seconds with intervals of aerosol delivery at 10LPM for several durations. Aerosol delivery was more efficient when the nebuliser was placed at the nasal cannula (~23%), compared to when placed at the wet side of the humidifier (~19%).

Phasic aerosol delivery was tested using the same gas flow controller set-up. The nebuliser was placed at the nasal cannula. A significantly greater tracheal dose was observed with phasic delivery (~58%), compared to without phasic delivery (~23%). Statistically significant differences were observed across all aerosol durations.

Optimal aerosol delivery was achieved when the nebuliser was placed at the nasal cannula, and when aerosol was delivered in a phasic manner. A nebuliser of low droplet size was associated with a marginal increase in tracheal dose. Phasic delivery method yielded high aerosol efficiency.

The system as described may be a discrete standalone assembly, interposed between a remote gas supply, e.g. wall gas or turbine, and the humidifier and patient circuit. Alternatively, it may be integrated in a ventilator system wherein aerosol generator operation and cycle time can be adjusted independently or by a pre-determined algorithm. Such a system provides ease of use benefits to the user.

It will be appreciated that the invention provides for very effective delivery of aerosol for treatment of a patient who is undergoing high flow gas treatment, despite the challenges that such gas flow presents. It achieves the aerosol treatment with only minimal effect on the gas flow for provision of oxygen and elimination of CO2 dead spaces.

The invention is not limited to the embodiments described but may be varied in construction and detail within the scope of the claims. For example, the flow line may comprise two or more branches, preferably unidirectional towards the nasal cannula. Components described for some embodiments may be used in other embodiments as will be apparent to those skilled in the art.

## Claims

1. A gas therapy system (1) comprising:
a flow line (3),
a gas source or a link (6, 7) to a gas source,
a nebulizer (4) or a link to a nebulizer for aerosol delivery,
a patient interface (2) or a coupler for connection to a patient interface, and
a controller (10) configured to modulate gas flow and aerosol delivery in real time,
wherein the controller (10) is configured to:
change (24, 27) gas flow rate between at least one upper level and at least one lower level, and to
dynamically stop or reduce (26) aerosol delivery during gas flow at the upper level, and to
dynamically activate or increase (25) aerosol delivery during intervals of gas flow at the lower level,
wherein:
the system comprises a sensor to detect patient inhalation and/or exhalation, the controller is configured to provide aerosol delivery primarily during inhalation,
the controller is configured to activate aerosol generation during said interval a time duration Δ2 after switching to the lower gas flow level, and the time duration Δ2 has a value in the range of 0.01 seconds to 3.0 seconds, and
the controller is configured to de-activate the aerosol generation a time duration Δ3 before end of inhalation, and Δ3 has a value in the range of 0.01 seconds to 3.0 seconds.

2. A gas therapy system as claimed in claim 1, wherein said sensor is a pressure sensor.

3. A gas therapy system as claimed in any preceding claim, wherein the sensor includes one or more selected from a movement detector, a diaphragm displacement sensor, an ultra-wideband sensor, an impedance plethysmography sensor, a respiratory inductance plethysmography sensor, and an elastomeric plethysmography sensor, a temperature sensing device, and a humidity sensing device.

4. A gas therapy system as claimed in any of claims 1 to 3, wherein the sensor includes a gas or volatiles sensing device.

5. A gas therapy system as claimed in claim 4, wherein the sensor is adapted to detect carbon dioxide or a tracer gas or volatile delivered in the gas flow.

6. A gas therapy system as claimed in any preceding claim, wherein the system is adapted to generate a bolus of aerosol within the flow line for optimal aerosol delivery during inhalation, preferably in advance of and including the start of peak inhalation.

7. A gas therapy system as claimed in any preceding claim, wherein the controller (10) is configured to determine an optimal duration for the interval by calculating a value for internal circuit volume between the aerosol generator and the end of the patient interface or coupler divided by the gas flow rate, to determine time required for aerosol generation through to delivery at the patient interface.

8. A gas therapy system as claimed in any preceding claim, wherein the controller (10) is configured to dynamically calculate optimal aerosol generation and/or gas flow parameters dependent on location in the flow line of the aerosol generator.

9. A gas therapy system as claimed in any preceding claim, wherein the controller (10) is configured to perform modulation of oxygen supplied during the intervals to maintain a desired patient blood oxygenation.

10. A gas therapy system as claimed in claim 9, wherein the system includes a real time pulse oximeter and an oxygen regulator to perform said modulation.

11. A gas therapy system as claimed in any preceding claim, wherein the system comprises a humidifier (5) located adjacent to the aerosol generator.

12. A gas therapy system as claimed in any preceding claim, wherein the aerosol generator (4) is placed adjacent to or in the patient interface (2) or coupler.

13. A gas therapy system as claimed in any preceding claim, wherein the duration of said intervals is in the range of 5 seconds to 20 seconds, and optionally said duration is in the range of 8 seconds to 15 seconds.

14. A gas therapy system as claimed in any preceding claim, wherein the system comprises a flow sensor in the flow line and the controller is configured to use output signals from the flow sensor to monitor gas flow from the gas source and to use said flow monitoring when controlling gas flow rate.

## Patentansprüche

1. Gastherapiesystem (1), umfassend:
eine Strömungsleitung (3),
eine Gasquelle oder eine Anbindung (6, 7) an eine Gasquelle,
einen Vernebler (4) oder eine Anbindung an einen Vernebler zur Aerosolzufuhr,
eine Patientenschnittstelle (2) oder eine Kupplung zur Verbindung mit einer Patientenschnittstelle, und
eine Steuereinheit (10), die dazu konfiguriert ist, den Gasstrom und die Aerosolzufuhr in Echtzeit zu modulieren,
wobei die Steuereinheit (10) dazu konfiguriert ist:
den Gasvolumenstrom zwischen mindestens einem oberen Niveau und mindestens einem unteren Niveau zu ändern (24, 27), und
die Aerosolzufuhr während des Gasstroms auf dem oberen Niveau dynamisch anzuhalten oder zu verringern (26), und
die Aerosolzufuhr während Intervallen des Gasstroms auf dem unteren Niveau dynamisch zu aktivieren oder zu erhöhen (25),
wobei:
das System einen Sensor zum Erkennen der Patienteninhalation und/oder -exhalation umfasst, die Steuereinheit dazu konfiguriert ist, die Aerosolzufuhr vorwiegend während der Inhalation bereitzustellen,
die Steuereinheit dazu konfiguriert ist, die Aerosolerzeugung während des Intervalls eine Zeitdauer Δ2 nach dem Wechsel zu dem unteren Gasstromniveau zu aktivieren, und die Zeitdauer Δ2 einen Wert im Bereich von 0,01 Sekunden bis 3,0 Sekunden aufweist, und
die Steuereinheit dazu konfiguriert ist, die Aerosolerzeugung eine Zeitdauer Δ3 vor dem Ende der Inhalation zu deaktivieren, und Δ3 einen Wert im Bereich von 0,01 Sekunden bis 3,0 Sekunden aufweist.

2. Gastherapiesystem nach Anspruch 1, wobei es sich bei dem Sensor um einen Drucksensor handelt.

3. Gastherapiesystem nach einem der vorangehenden Ansprüche, wobei der Sensor ein oder mehrere aus Folgenden ausgewählte umfasst: einem Bewegungsdetektor, einem Membranauslenkungssensor, einem Ultrabreitbandsensor, einem Impedanz-Plethysmographiesensor, einem Atemwegsinduktivitäts-Plethysmographiesensor und einem Elastomerplethysmographiesensor, einer Temperaturabfühlvorrichtung und einer Feuchtigkeitsabfühlvorrichtung.

4. Gastherapiesystem nach einem der Ansprüche 1 bis 3, wobei der Sensor eine Abfühlvorrichtung für Gas oder flüchtige Stoffe umfasst.

5. Gastherapiesystem nach Anspruch 4, wobei der Sensor dazu angepasst ist, Kohlendioxid oder ein Tracergas oder einen flüchtigen Stoff, das bzw. der in den Gasstrom zugeführt wurde, zu erkennen.

6. Gastherapiesystem nach einem der vorangehenden Ansprüche, wobei das System dazu angepasst ist, zur optimalen Aerosolzufuhr während der Inhalation, vorzugsweise vor dem Beginn der Spitzeninhalation und diesen einschließend einen Bolus von Aerosol innerhalb der Strömungsleitung zu erzeugen.

7. Gastherapiesystem nach einem der vorangehenden Ansprüche, wobei die Steuereinheit (10) dazu konfiguriert ist, eine optimale Dauer für das Intervall zu bestimmen, indem sie einen Wert für das interne Kreislaufvolumen zwischen dem Aerosolerzeuger und dem Ende der Patientenschnittstelle oder der Kupplung geteilt durch den Gasvolumenstrom berechnet, um die Zeit zu bestimmen, die für die Aerosolerzeugung bis zur Zufuhr an der Patientenschnittstelle benötigt wird.

8. Gastherapiesystem nach einem der vorangehenden Ansprüche, wobei die Steuereinheit (10) dazu konfiguriert ist, optimale Aerosolerzeugungs- und/oder Gasstromparameter abhängig von der Stelle in der Strömungsleitung des Aersolerzeugers dynamisch zu berechnen.

9. Gastherapiesystem nach einem der vorangehenden Ansprüche, wobei die Steuereinheit (10) dazu konfiguriert ist, eine Modulation von während der Intervalle zugeführtem Sauerstoff auszuführen, um eine gewünschte Patientenblutoxygenierung aufrechtzuerhalten.

10. Gastherapiesystem nach Anspruch 9, wobei das System ein Echtzeit-Pulsoximeter und einen Sauerstoffregler zum Ausführen der Modulation umfasst.

11. Gastherapiesystem nach einem der vorangehenden Ansprüche, wobei das System einen Befeuchter (5) umfasst, der sich dem Aerosolerzeuger benachbart befindet.

12. Gastherapiesystem nach einem der vorangehenden Ansprüche, wobei der Aerosolerzeuger (4) der Patientenschnittstelle (2) oder der Kupplung benachbart oder darin platziert ist.

13. Gastherapiesystem nach einem der vorangehenden Ansprüche, wobei die Dauer der Intervalle im Bereich von 5 Sekunden bis 20 Sekunden und optional die Dauer im Bereich von 8 Sekunden bis 15 Sekunden liegt.

14. Gastherapiesystem nach einem der vorangehenden Ansprüche, wobei das System einen Strömungssensor in der Strömungsleitung umfasst und die Steuereinheit dazu konfiguriert ist, Ausgangssignale von dem Strömungssensor zu verwenden, um den Gasstrom von der Gasquelle zu überwachen und die Stromüberwachung beim Steuern des Gasvolumenstroms zu verwenden.

## Revendications

1. Système de thérapie par gaz (1) comprenant :
une conduite d'écoulement (3),
une source de gaz ou une liaison (6, 7) à une source de gaz,
un nébuliseur (4) ou une liaison à un nébuliseur pour la délivrance d'aérosol,
une interface de patient (2) ou un coupleur pour une connexion à une interface de patient, et
un dispositif de commande (10) configuré pour moduler l'écoulement de gaz et la délivrance d'aérosol en temps réel,
dans lequel le dispositif de commande (10) est configuré pour :
changer (24, 27) le débit de gaz entre au moins un niveau supérieur et au moins un niveau inférieur, et pour
interrompre ou réduire de manière dynamique (26) la délivrance d'aérosol pendant l'écoulement de gaz au niveau supérieur, et pour
activer ou augmenter de manière dynamique (25) la délivrance d'aérosol pendant les intervalles d'écoulement de gaz au niveau inférieur,
dans lequel :
le système comprend un capteur pour détecter l'inhalation et/ou l'exhalation du patient, le dispositif de commande est configuré pour fournir une délivrance d'aérosol principalement pendant l'inhalation,
le dispositif de commande est configuré pour activer une génération d'aérosol pendant ledit intervalle pendant une durée Δ2 après la commutation vers le niveau inférieur d'écoulement de gaz, et la durée Δ2 a une valeur comprise dans la plage de 0,01 seconde à 3,0 secondes, et
le dispositif de commande est configuré pour désactiver la génération d'aérosol pendant une durée Δ3 avant la fin de l'inhalation, et Δ3 a une valeur comprise dans la plage de 0,01 seconde à 3,0 secondes.

2. Système de thérapie par gaz selon la revendication 1, dans lequel ledit capteur est un capteur de pression.

3. Système de thérapie par gaz selon l'une quelconque des revendications précédentes, dans lequel le capteur inclut un ou plusieurs éléments choisis parmi un capteur de mouvement, un capteur de déplacement de diaphragme, un capteur à bande ultra-large, un capteur par pléthysmographie à impédance, un capteur par pléthysmographie d'inductance respiratoire, et un capteur par pléthysmographie elastomérique, un dispositif de détection de température, et un dispositif de détection d'humidité.

4. Système de thérapie par gaz selon l'une quelconque des revendications 1 à 3, dans lequel le capteur inclut un dispositif de détection de gaz ou de substances volatiles.

5. Système de thérapie par gaz selon la revendication 4, dans lequel le capteur est conçu pour détecter le dioxyde de carbone ou un gaz traceur ou une substance volatile délivré dans l'écoulement de gaz.

6. Système de thérapie par gaz selon l'une quelconque des revendications précédentes, dans lequel le système est conçu pour générer un bol d'aérosol au sein de la conduite d'écoulement pour une délivrance d'aérosol optimale pendant l'inhalation, de préférence avant et incluant le début de l'inhalation maximale.

7. Système de thérapie par gaz selon l'une quelconque des revendications précédentes, dans lequel le dispositif de commande (10) est configuré pour déterminer une durée optimale pour l'intervalle en calculant une valeur pour le volume de circuit interne entre le générateur d'aérosol et l'extrémité de l'interface de patient ou du coupleur divisé par le débit de gaz, pour déterminer le temps requis pour la génération d'aérosol jusqu'à la délivrance au niveau de l'interface de patient.

8. Système de thérapie par gaz selon l'une quelconque des revendications précédentes, dans lequel le dispositif de commande (10) est configuré pour calculer de manière dynamique des paramètres optimaux de génération d'aérosol et/ou d'écoulement de gaz en fonction de l'emplacement dans la conduite d'écoulement du générateur d'aérosol.

9. Système de thérapie par gaz selon l'une quelconque des revendications précédentes, dans lequel le dispositif de commande (10) est configuré pour réaliser la modulation de l'oxygène apporté pendant les intervalles pour maintenir une oxygénation du sang du patient souhaitée.

10. Système de thérapie par gaz selon la revendication 9, dans lequel le système inclut un oxymètre de pouls en temps réel et un régulateur d'oxygène pour réaliser ladite modulation.

11. Système de thérapie par gaz selon l'une quelconque des revendications précédentes, dans lequel le système comprend un humidificateur (5) situé à côté du générateur d'aérosol.

12. Système de thérapie par gaz selon l'une quelconque des revendications précédentes, dans lequel le générateur d'aérosol (4) est placé à côté de ou dans l'interface de patient (2) ou le coupleur.

13. Système de thérapie par gaz selon l'une quelconque des revendications précédentes, dans lequel la durée desdits intervalles est comprise dans la plage de 5 secondes à 20 secondes, et éventuellement la durée est comprise dans la plage de 8 secondes à 15 secondes.

14. Système de thérapie par gaz selon l'une quelconque des revendications précédentes, dans lequel le système comprend un capteur de débit dans la conduite d'écoulement et le dispositif de commande est configuré pour utiliser des signaux de sortie provenant du capteur de débit pour surveiller l'écoulement de gaz depuis la source de gaz et pour utiliser ladite surveillance d'écoulement pendant la commande du débit de gaz.
